# EUROPEAN PATENT SPECIFICATION

(11) **EP 4 397 960 B1**
(45) Date of publication and mention of the grant of the patent: **25.11.2026**
(21) Application number: 24150896.9
(22) Date of filing: 09.01.2024
(51) Int. Cl.: G01N 21/31, G01N 21/47, A61B 5/145, A61B 5/1455, G01J 3/02, G01J 3/10, G01J 3/42, A61B 5/00

(54) **APPARATUS AND METHOD FOR ESTIMATING AN ANTIOXIDANT LEVEL**
VORRICHTUNG UND VERFAHREN ZUR SCHÄTZUNG EINES ANTIOXIDATIONSMITTELGEHALTS
APPAREIL ET PROCÉDÉ D'ESTIMATION D'UN NIVEAU D'ANTIOXYDANT

(30) Priority: 09.01.2023 KR 20230002877
(43) Date of publication of application: 10.07.2024
(73) Proprietor: Samsung Electronics Co., Ltd., Suwon-si, Gyeonggi-do 16677 (KR)
(72) Inventor: Jung, Myoung Hoon, Suwon-si, Gyeonggi-do (KR); Park, Jin Young, Suwon-si, Gyeonggi-do (KR); Eom, Kun Sun, Suwon-si, Gyeonggi-do (KR); Hwang, Jeong Eun, Suwon-si, Gyeonggi-do (KR); Kim, Yoon Jae, Suwon-si, Gyeonggi-do (KR); Moon, Hyun Seok, Suwon-si, Gyeonggi-do (KR)
(74) Representative: Grünecker Patent- und Rechtsanwälte PartG mbB

(56) References cited:
- EP-A1- 4 179 963
- EP-A1- 4 186 415
- US-A1- 2009 306 521
- US-A1- 2017 035 308
- US-A1- 2017 303 830
- US-A1- 2020 029 873

## Description

### BACKGROUND

### 1. Field

The disclosure relates to an apparatus and method for estimating antioxidant levels.

### 2. Description of Related Art

Reactive oxygen species (ROS) act as an important biological defense factor against free radicals similar to the protection offered by white blood cells that protect a body against infections. However, it has been known that excessive generation of ROS in the body may lead to various tissue diseases. Common factors that cause generation of ROS include stress, alcohol, peroxides, medicine, and the like. The ROS produced by these factors may cause cranial nerve diseases, circulatory diseases, cancer, digestive tract diseases, liver diseases, arteriosclerosis, renal diseases, diabetes, aging, and the like. Living organisms have a series of antioxidant defense systems to protect against oxygen toxicity. Antioxidants play a role in the normal operation of such antioxidant defense systems. Antioxidants may include, for example, vitamin E, vitamin C, carotenoid, and flavonoid.
US 2020/029873 A1 refers to an antioxidant sensor, antioxidant signal obtaining method, and antioxidant level determining method. The antioxidant sensor includes a first light source configured to emit light having a first wavelength onto an object, a second light source configured to emit light having a second wavelength onto the object, a third light source configured to emit light having a third wavelength onto the object. The first wavelength, the second wavelength, and the third wavelength are different from each other. A light receiver is configured to receive light reflected or scattered from the object. A processor is configured to obtain a hemoglobin index by driving the first light source and the second light source, and to obtain an antioxidant signal of the object by driving the third light source based on the obtained hemoglobin index satisfying a condition.
US 2017/035308 A1 refers to a noninvasive system for measurement of heart rate and other heart-related characteristics a photoplethysmogram, PPG, signal obtained from a tissue is divided into several feature waveforms, each corresponding to a PPG window of a particular length. Conditioned features, containing frequency components specific to heart-related events, are derived from the features by modulating a carrier kernel with such features. The conditioned features are computationally collided with one or more Zyotons that are co-dependent with the conditioned features. For each conditioned feature, one or more collisions selectively amplify frequency components in features sourced from PPG, and respective energy change values are obtained from such amplified energy portions. The resulting energy change values are analyzed to determine a smallest time-window likely containing heart rate and other heart-related events in the PPG data stream.
US 2017/303830 A1 refers to systems, devices and methods for noninvasive analysis of tissue, by irradiating a surface of the tissue with infrared radiation such that an interaction of the radiation with a component of the tissue other than water in two spectral bands is substantially identical, measuring an intensity of the radiation that emerges from the tissue in each of the spectral bands, determining change in at least one of shape and intensity of signals received by the at least one radiation detector, calculating a relative absorption by the tissue of radiation in one of the first and second spectral bands relative to absorption by the tissue of radiation in the other of the first and second spectral bands, and determining concentration of a predetermined substance, in accordance with the calculated relative absorption and in accordance with determined change in the received signal.
US 2009/306521 A1 refers to noninvasive measurement of carotenoid antioxidants and similar chemical compounds in biological tissue such as living skin. A method and an apparatus provide a non-invasive, rapid, accurate, and safe determination of carotenoid levels which in turn can provide diagnostic information of the antioxidant status of tissue. Reflection spectroscopy is used to measure the concentrations of carotenoids and similar substances in tissue. White light is directed upon the area of tissue that is of interest. A small fraction of diffusively scattered light is collected and measured. The tissue is pressured to temporarily squeeze blood out of the measured tissue volume while the reflection spectrum is continuously monitored, displayed, and analyzed in near real time. After an optimal time period of typically 15 seconds, the influence of the dominating hemoglobin and oxyhemoglobin tissue absorptions on the reflection spectra are minimized.
EP 4 179 963 A1 is prior art under Article 54(3) EPC, and refers to an electronic device and method of estimating bio-information using same.
EP 4 186 415 A1 is prior art under Article 54(3) EPC, and refers to an electronic device and method of estimating bio-information using the same.

### SUMMARY

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

Additional aspects will be set forth in part in the description which follows and, in part, will be apparent from the description, or may be learned by practice of the presented embodiments.

The invention is defined in the independent claims 1, 10 and 12. Preferred embodiments are specified in the dependent claims.

According to an aspect of an example embodiment, an apparatus for estimating an antioxidant level, comprises : a sensor comprising at least one light source configured to emit light to an object, and at least one first detector configured to detect at least one first light signal scattered or reflected by the object, and at least one second detector configured to detect at least one second light signal scattered or reflected by the object, each of the at least one first light signal having a first light path from the object and each of the at least one second light signal having a second light path from the object that is different from the first light path; and a processor configured to:based on the at least one first light signal being detected, obtain a first absorbance by combining first light quantities of the first light path for each wavelength based on the at least one first light signal, wherein obtaining the first absorbance comprises transforming each of the at least one first light signal into a first logarithmic domain and combining the transformed at least one first light signal to obtain the first absorbance, based on the at least one second light signal being detected, obtain a second absorbance by combining second light quantities of the second light path for each wavelength based on the at least one second light signal, wherein obtaining the second absorbance comprises transforming each of the at least one second light signal into a second logarithmic domain and combining the transformed at least one second light signal to obtain the second absorbance, and estimate the antioxidant level based on a difference between the first absorbance and the second absorbance for each wavelength without performing a calibration process for measuring the light emitted by the at least one light source.

The at least one light source may be disposed at a center of the sensor, the at least one first detector may include a plurality of first detectors arranged in a first concentric circular shape at a first distance from the center of the sensor, and the at least one second detector may include a plurality of second detectors arranged in a second concentric circular shape at a second distance from the center of the sensor.

The at least one first detector may be disposed at a center of the sensor, the at least one light source may include a plurality of light sources arranged in a first concentric circular shape at a first distance from the center of the sensor, and the at least one second detector may include a plurality of second detectors arranged in a second concentric circular shape at a second distance from the first concentric circular shape.

The at least one second detector may be disposed at a center of the sensor, the at least one light source may include a plurality of light sources arranged in a second concentric circular shape that is at a second distance from the center of the sensor, and the at least one first detector arranged in a first concentric circular shape at a first distance from the second concentric circular shape.

The at least one light source have a wavelength in a range from 400 nm to 600 nm.

The apparatus may further include at least one partition wall disposed between the at least one light source and the at least one first detector or between the at least one light source and the at least one second detector.

A difference between the first light path and the second light path may be between 2 mm and 15 mm.

The processor may be further configured to combine the transformed at least one first light signal and combine the at least one second light signal using at least one of an arithmetic mean, a harmonic mean, or a geometric mean.

The processor may be further configured to: determine an antioxidant peak based on the difference between the first absorbance and the second absorbance for a plurality of wavelengths, and obtain the antioxidant level based on the antioxidant peak.

According to an aspect of an example embodiment, a method of operating a device worn on a body part of a user for estimating an antioxidant level of the user, comprises : emitting, by a light source, light to the body part;detecting, by a first detector spaced apart from the light source, at least one first light signal scattered or reflected by the body part from the light emitted by the light source, each of the at least one first light signal having a first light path from the object;detecting, by a second detector spaced further apart from the light source than the first detector, at least one second light signal scattered or reflected by the body part from the light emitted by the light source, each of the at least one second light signal having a second light path from the object that is different from the first light path;based on the at least one first light signal being detected, obtaining, by a processor, a first absorbance by combining first light quantities of the first light path for each wavelength based on the at least one first light signal, wherein obtaining the first absorbance comprises transforming each of the at least one first light signal into a first logarithmic domain and combining the transformed at least one first light signal to obtain the first absorbance;based on the at least one second light signal being detected, obtaining, by the processor, a second absorbance by combining second light quantities of the second light path for each wavelength based on the at least one second light signal,wherein obtaining the second absorbance comprises transforming each of the at least one second light signal into a second logarithmic domain and combining the transformed at least one second light signal to obtain the second absorbance; and estimating, by the processor, the antioxidant level of the user based on a difference between the first absorbance and the second absorbance for each wavelength without performing a calibration process for measuring the light emitted by the light source.

The estimating may include determining an antioxidant peak based on the difference between the first absorbance and the second absorbance for a plurality of wavelengths, and obtaining the antioxidant level based on the antioxidant peak.

The device may be a smart watch configured to be worn on a wrist of the user.

The processor is configured to estimate the antioxidant level without prior calibration of the light source.

The device may further include partition walls disposed between the light source and each of the first detector and the second detector.

The second detector may be spaced between 2 mm and 15 mm further apart from the light source than the first detector.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a block diagram illustrating an apparatus for estimating bio-information;
FIGS. 2A, 2B, and 2C are diagrams illustrating various examples of the structure of a sensor;
FIGS. 3A and 3B are diagrams for explaining an example of transforming a plurality of light signals detected by a plurality of detector into a logarithmic domain and combining the transformed light signals;
FIG. 4 is a graph showing a deviation between sensors when distance information is averaged and when distance information is not averaged;
FIG. 5 is a block diagram illustrating an apparatus for estimating bio-information;
FIG. 6 is a flowchart illustrating a method of estimating bio-information; and
FIGS. 7, 8, and 9 are diagrams illustrating structures of an electronic device.

### DETAILED DESCRIPTION

Details of examples and embodiments are included in the following detailed description and drawings. Advantages and features of the disclosure, and a method of achieving the same will be more clearly understood from the following embodiments and examples described in detail with reference to the accompanying drawings. Throughout the drawings and the detailed description, unless otherwise described, the same drawing reference numerals will be understood to refer to the same elements, features, and structures. In the drawings, the relative size and depiction of these elements may be exaggerated for clarity, illustration, and convenience.

It will be understood that, although the terms first, second, etc. may be used herein to describe various elements, these elements should not be limited by these terms. These terms may be used to distinguish one element from another. Also, the singular forms are intended to include the plural forms as well, unless the context clearly indicates otherwise. It will be further understood that when an element is referred to as "comprising" another element, the element is intended not to exclude one or more other elements, but to further include one or more other elements, unless explicitly described to the contrary. In the following description, terms such as "unit" and "module" indicate a unit for processing at least one function or operation and they may be implemented by using hardware, software, or a combination thereof.

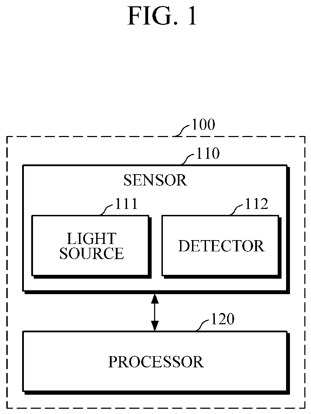

FIG. 1 is a block diagram illustrating an apparatus for estimating bio-information.

Referring to FIG. 1, an apparatus 100 for estimating an antioxidant level includes a sensor 110 and a processor 120.

The sensor 110 is configured to measure two or more light signals having different light paths from an object of the user. For example, the object may include a palm or sole in which an epidermal layer is thick, an area in which venous blood or capillary blood is located or which is adjacent to the radial artery, and other peripheral parts of the human body, such as fingers, toes, or earlobes, which have a high density of blood vessels, and body parts such as wrists, or inner ears.

The sensor 110 includes a light source 111 configured to emit light to the object and a detector 112 configured to detect light reflected from the object. The light source 111 may include a light emitting diode (LED), a laser diode, a phosphor, or the like. The wavelength of the light source may range from 400 nm to 600 nm. The light source 111 may be provided in singular or plural numbers (e.g., three or more) and each may emit light of a different wavelength. The number, wavelength band, and the like of the light source 11 may be vary without limitation according to the type of bio-information, the size of form factor of the apparatus 100, and the like. A plurality of light sources 111 may be sequentially driven in a time-division manner, or simultaneously driven. Alternatively, a group of a predetermined number of light sources may be set as a unit group, the unit groups of the light sources are driven sequentially while the light sources in each group are simultaneously driven. The detector 112 may include, for example, a photodiode, a photo transistor, or the like, and one or more detectors 112 may be provided. However, this embodiment is not limited thereto, and the detector 112 may include a complementary metal-oxide semiconductor (CMOS) image sensor, a charge-coupled device (CCD) image sensor, or the like.

The light source 111 and the detector 112 are arranged to have two or more light paths. For example, a detector (hereinafter referred to as a "first detector") may be disposed at a first distance from the light source 111 to have a first light path, and a detector (hereinafter referred to as a "second detector") may be disposed at a second distance from the light source 111 to have a second light path. The number and arrangement of the light sources and the detectors are not particularly limited. The second detector may be disposed 2 mm to 15 mm further away from the light source 111 than the first detector. A difference between the light paths may be 2 mm or greater and 15 mm or less. This embodiment is not limited thereto, and the difference between the light paths may vary without limitations according to the size of form factor, the contact area of the object, and the like.

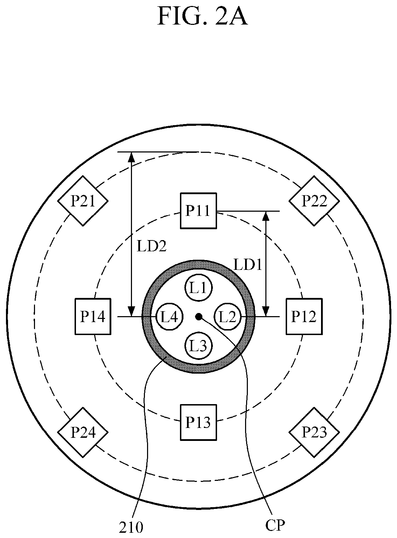

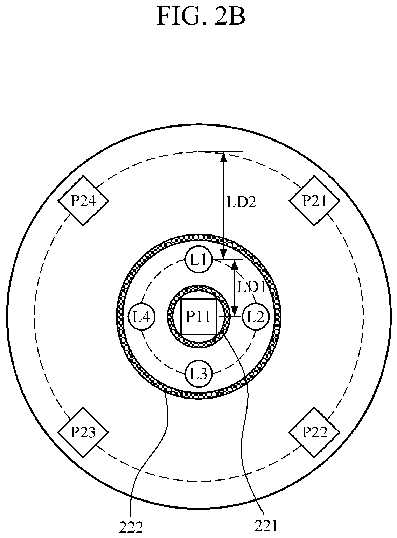

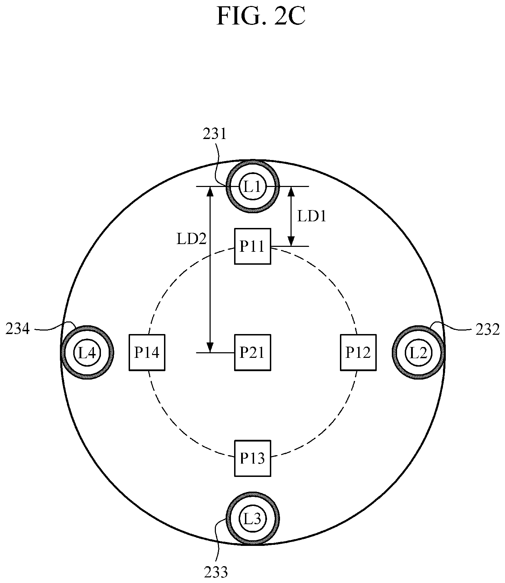

FIGS. 2A, 2B, and 2C are diagrams illustrating various examples of the structure of the sensor 110.

Referring to FIG. 2A, light sources L1, L2, L3, and L4 may be disposed at the center of the sensor 110, and first detectors P11, P12, P13, and P14 and second detectors P21, P22, P23, and P24 may be disposed in a concentric circular shape at a first distance LD1 and a second distance LD2, respectively, from the center CP of the light sources L1, L2, L3, and L4. The center CP of the light sources L1, L2, L3, and L4 may mean the center of the light sources when there is one light source, and may mean the center of a space formed by a plurality of light sources when there are a plurality of light sources.

Referring to FIG. 2B, the first detector P11 may be disposed at the center of the sensor 110, and the light sources L1, L2, L3, and L4 may be disposed in a concentric circular shape at the first distance LD1 from the center of the first detector P11. The second detectors P21, P22, P23, and P24 may be disposed in a concentric circular shape at the second distance LD2 from the light sources L1, L2, L3, and L4. That is, the distance between the concentric circle on which the light sources L1, L2, L3, and L4 are disposed and the concentric circle on which the second detectors P21, P22, P23, and P24 may be the second distance LD2.

Referring to FIG. 2C, the second detector P21 may be disposed at the center of the sensor 110, and the light sources L1, L2, L3, and L4 may be disposed in a concentric circular shape at the second distance LD2 from the center of the second detector P21. Also, the first detectors P11, P12, P13, and P14 may be disposed in a concentric circular shape at the first distance LD1 from the light sources L1, L2, L3, and L4 in an inward direction.

Referring to FIGS. 2A, 2B, and 2C, the sensor 110 may further include partition walls 210, 221, 222, 231, 233, and 234 to prevent light emitted from the light sources L1, L2, L3, and L4 from being directly incident onto the first detectors P11, P12, P13, and P14 and the second detectors P21, P22, P23, and P24. The partition walls may be have a ring shape that surrounds the entire light sources L1, L2, L3, and L4 (partition wall 210 in FIG. 2A and partition wall 222 in FIG. 2B), a ring shape that surrounds each of the light sources L1, L2, L3, and L4 (partition walls 231, 232, 233, and 234 in FIG. 2C), a ring shape that surrounds the detector P11 (partition wall 221 in FIG. 2B), or the like. However, the shape of the partition wall is not limited to the ring shape, and may be modified in various shapes, such as a triangular shape, a rectangular shape, or the like, and may be omitted as necessary in consideration of the arrangement of the sensor 110, the form factor, the type of bio-information to be estimated, the purpose of estimation, required accuracy, etc.

Referring back to FIG. 1, the processor 120 may be electrically connected to the sensor 110 or connected thereto via wireless communications and control the sensor 110. The processor 120 may drive the sensor 110 and the light source 111 upon a user's request for estimating antioxidant activity, and may obtain a first light signal and a second light source by driving, respectively, the first detector 112 on a first light path and the second detector 112 on a second light path while the light source 111 is turned on. In this case, the first detector 112 and the second detector 112 may be simultaneously driven, or may be sequentially driven (first detector->second detector or second detector->first detector). In a case where a plurality of light sources 111 are provided, the light sources of each wavelength are sequentially driven in a time-division manner, and a first light signal and a second signal may be obtained for each wavelength.

The processor 120 obtains a first absorbance using the first light signal and a second absorbance using the second light signal for each wavelength, and obtain an antioxidant level based on the first absorbance and the second absorbance. For example, the antioxidant level may be estimated based on a difference between the first absorbance and the second absorbance for each wavelength. As described below, since an antioxidant level is estimated using a difference in absorbance of light signals having different light paths, there is no need to perform calibration of the light sources used for obtaining the amount of light emitted initially before estimating the antioxidant level.

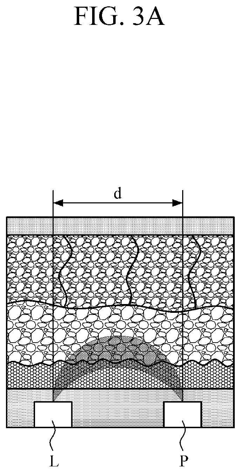

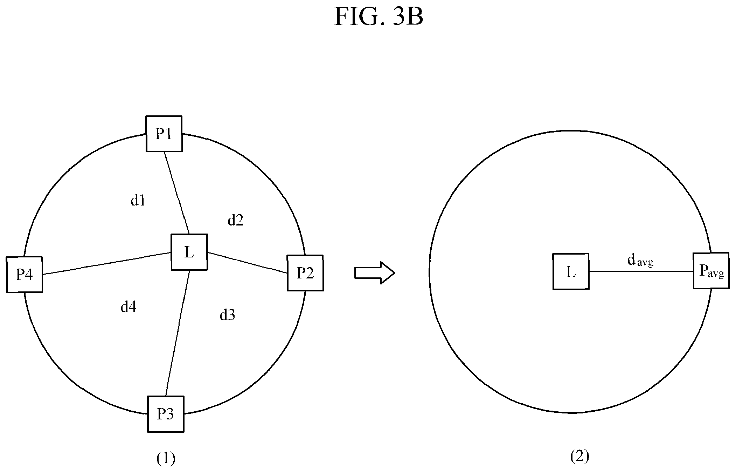

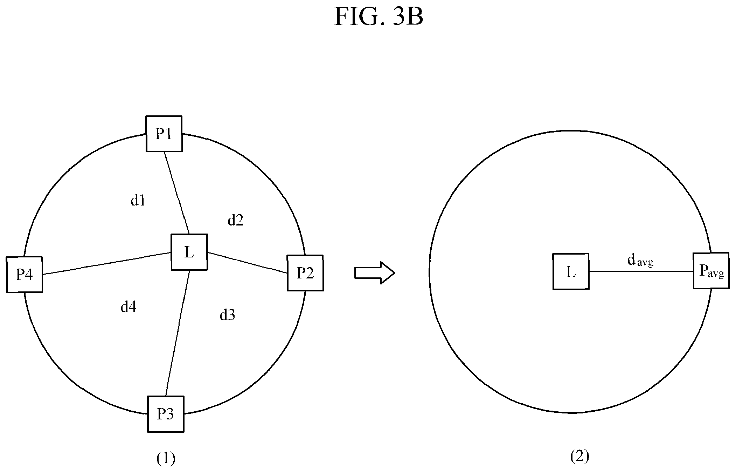

FIGS. 3A and 3B are diagrams for explaining an example of transforming a plurality of light signals detected by a plurality of detector into a logarithmic domain and combining the transformed light signals.

Referring to FIG. 3A, generally, when light emitted from a light source L is scattered or reflected by an object and reaches a detector P, the amount of light measured at the detector P may be represented by Equation 1 based on the Lambert-Beer's law as shown below. $I = {I}_{0} {10}^{- εcd}$ (1)

In Equation 1, / denotes the amount of light detected by the detector. *I*₀ denotes the amount of light emitted from the light source, which may be the amount of light measured through a standard reflector at the time of calibration. *ε* denotes an absorption coefficient of an antioxidant component, c denotes the concentration of the antioxidant component, and *d* denotes a distance between the light source L and the detector P.

With respect to the light source of each wavelength, the processor 120 may represent each of a first light quantity of the first light signal detected by the first detector and a second light quantity of the second light signal measured by the second detector as a linear relationship equation of Equation 1.

The processor 120 transforms a linear domain into a logarithmic domain by applying a logarithmic operation to the linear relationship equation for each of the first light quantity and the second light quantity, which may be represented as shown below in Equation 2. In the logarithmic operation, the base of the logarithm may be 10 or a natural constant e, and is not limited thereto. Also, a nonlinear function, a regression equation, an empirical equation may be used instead of the logarithmic operation depending on the characteristics of the sensor to be fabricated, and the embodiment is not limited thereto. $log I = {logI}_{0} - εcd$ (2)

The processor 120 obtains the first absorbance and the second absorbance by combining the first light quantities transformed into a logarithmic domain and the second light quantities transformed into a logarithmic domain, respectively. In the case where, as shown in (1) of FIG. 3B, four detectors P1, P2, P3, and P4 are disposed at distances d1, d2, d3, and d4, respectively, from the light source L, light quantities, each transformed into a logarithmic domain through Equation 2, may be combined to be averaged as a light quantity detected by one detector P_{avg} at an average distance d_{avg} from the light source L as shown in (2) of FIG. 3B. In this way, a deviation of distances d1, d2, d3, and d4 of the plurality of detectors P1, P2, P3, and P4 from the light source L of a specific wavelength may be canceled. Equation 3 below is an example of combining the light quantities transformed into the logarithmic domain of the four detectors P1, P2, P3, and P4. ${I}_{fm} = f \left({logI}_{m 1}, {logI}_{m 2}, {logI}_{m 3}, {logI}_{m 4}\right)$ (3)

log/ₘ₁, log/ₘ₂, log/ₘ₃, and log/ₘ₄ denote values obtained by transforming the light quantities obtained by the respective detectors P1, P2, P3, and P4 into a distance domain for a specific wavelength. *I_{fm}* denotes a value obtained by combining log/ₘ₁, log/ₘ₂, log/ₘ₃, and log/ₘ₄.

A combination function f may be one of arithmetic mean (see Equation 4 below), harmonic mean (see Equation 5 below), and geometric mean (see Equation 6 below), but is not limited thereto. The processor 120 may use an arithmetic mean filter, a harmonic mean filter, or a geometric mean filter to perform arithmetic mean, harmonic mean, or geometric mean. ${I}_{fm} = \frac{{logI}_{m 1} + {logI}_{m 2} + {logI}_{m 3} + {logI}_{m 4}}{4}$ (4) ${I}_{fm} = \frac{4 \left({logI}_{m 1}\times {logI}_{m 2}\times {logI}_{m 3}\times {logI}_{m 4}\right)}{{logI}_{m 1} + {logI}_{m 2} + {logI}_{m 3} + {logI}_{m 4}}$ (5) ${I}_{fm} = \sqrt[4]{{logI}_{m 1} \times {logI}_{m 2} \times {logI}_{m 3} \times {logI}_{m 4}}$ (6)

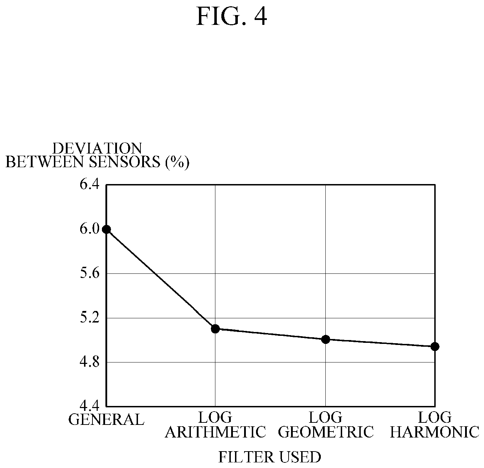

The arrangement deviation of a light source and a detector of the sensor may cause a change in the length of a light path between the light source and the detector, which may change the intensity of a measured signal and thus reduce the accuracy of bio-information estimation. In this example embodiment, a plurality of light quantities obtained by a plurality of detectors may be transformed into a logarithmic domain and then combined, thereby canceling a distance deviation. FIG. 4 is a graph showing a deviation between sensors when distance information is averaged and when distance information is not averaged. The X axis shows a general case (general) where distance information is not averaged, a case (log arithmetic) of arithmetic mean of distance information, a case (log geometric) of geometric mean of distance information, and a case (log harmonic) of harmonic mean of distance information, and the Y-axis represents a deviation between sensors in percentage. It can be seen that the deviation in the case of measuring bio-information by averaging distance information transformed into the logarithmic domain is maintained constant and is lower than the deviation in the general case where the distance information is not averaged.

The processor 120 obtains the first absorbance and the second absorbance by combining the first light quantities of the first light path and the second light quantities of the second light path for each wavelength, respectively, and obtain an antioxidant level using a difference between the first absorbance and the second absorbance. The difference between the first absorbance and the second absorbance is obtained by Equation 7 as shown below. ${A}_{2} - {A}_{1} = - {log}_{10} \frac{{I}_{1}}{{I}_{0}} + {log}_{10} \frac{{I}_{2}}{{I}_{0}} = εc Δ = {log}_{10} \frac{{I}_{1}}{{I}_{2}}$ (7)

*A₁* and *A₂* represent a first absorbance and a second absorbance of a light source of a specific wavelength. *l₁* and *l₂* denote a combined value of first quantities and a combined value of second quantities, respectively. *Δ* represents a difference between a first light path and a second light path. *l₀* denotes the amount of light emitted from the light source, and is eliminated in a relationship equation. Therefore, it is not necessary to perform a calibration process for measuring the amount of light emitted from the light source using a standard reflected wave, etc., so that user convenience can be improved. Also, accuracy may be improved by automatically correcting the change in light quantity due to temperature change, device deterioration and sensor fabrication characteristics by using light quantities on two or more light paths.

When the difference between the first absorbance and the second absorbance for each wavelength is obtained, the processor 120 may obtain an antioxidant level using the difference in absorbance. The processor 120 may obtain an antioxidant peak as a feature value. As shown below, Equation 8 is an example of obtaining an antioxidant peak by using three wavelengths and Equation 9 is an example of obtaining an antioxidant peak by using four wavelengths. $AO = {A}_{{λ}_{2}} - \left(\frac{{λ}_{3} - {λ}_{2}}{{λ}_{3} - {λ}_{1}}\right) \times {A}_{{λ}_{1}} - \left(\frac{{λ}_{2} - {λ}_{1}}{{λ}_{3} - {λ}_{1}}\right) \times {A}_{{λ}_{3}}$ (8)

Here, *AO* represents an antioxidant peak as an example of a feature value. *λ₁, λ₂,* and *λ₃* represent wavelengths, and *A_{λ1}, A_{λ2},* and *A_{λ3}* represent the difference in absorbance for each wavelength obtained through Equation 7 above. The wavelengths may be longer in the order of *A_{λ1}, A_{λ2},* and A_{λ3}. $AO = {aA}_{{λ}_{1}} + {bA}_{{λ}_{2}} + {cA}_{{λ}_{3}} + {dA}_{{λ}_{4}} + e$ (9)

Here, *A_{λ1}, A_{λ2}, A_{λ3}*, and *A_{λ4}* represent the difference in absorbance for each wavelength obtained through Equation 7 above. *a*, *b, c*, and *d* are predetermined coefficients, and e is a predetermined constant.

The processor 120 may obtain an antioxidant level based on an antioxidant peak obtained through Equations 8 and 9. In one example, an antioxidant peak value may be obtained as the antioxidant level. In another example, an antioxidant level may be obtained by inputting an antioxidant peak value into an antioxidant estimation model defined as shown in Equation 10. Equation 10 represents a linear equation, but is not limited thereto, and may be defined as a nonlinear equation, such as a logarithmic equation and an exponential equation. $Y = a \times AO + b$ (10)

Here, Y represents an antioxidant level, and a and b represent preset values.

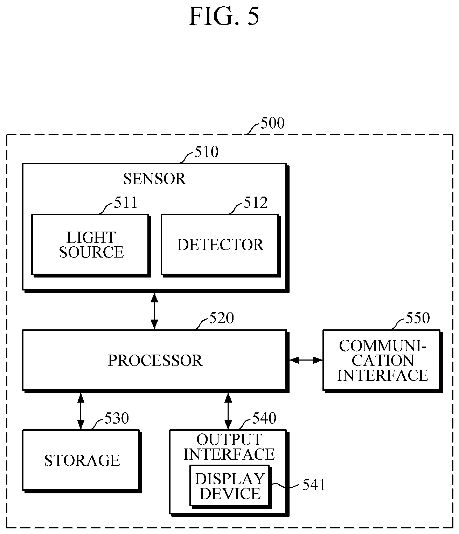

FIG. 5 is a block diagram illustrating an apparatus for estimating bio-information.

Referring to FIG. 5, an apparatus 500 for estimating bio-information may include a sensor 510, a processor 520, a storage (or memory) 530, an output interface 540, and a communication interface 550. The sensor 510 and the processor 520 are the same as the sensor 110 and the processor 120 of the example embodiment shown in FIG. 1, and thus detailed descriptions thereof will not be reiterated.

The storage 530 may store programs (e.g., application software) to be executed by the processor 520. The program may store one or more commands for performing various functions including the aforementioned bio-information estimation and input data and/or output data for the commands. For example, the storage 530 may store driving conditions of the sensor 510, a bio-information estimation model, user information (e.g., age, gender, height, weight, etc.), light signals measured by the sensor, an estimated bio-information value, and the like.

The storage 530 may include at least one type of storage medium of a flash memory type, a hard disk type, a multimedia card micro type, a card type memory (for example, secure digital (SD) or extreme digital (XD) memory), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk, but is not limited thereto.

The output interface 540 may output processing results of the processor 520 to a user. The output interface 540 may include a display device 541, provide a graphic user interface through the display device 541, and visually display the result of estimating bio-information on the graphic user interface. For example, the output interface 540 may display a graph showing change over time in antioxidant level, and may display a visual object movable on a time line above the graph. When the user moves the visual object to select an intended point in time, the output interface 540 may display bio-information, absorbance information, and the like at the selected point in time in response to the user's selection. In addition, when the bio-information falls outside a normal range, the output interface 540 may display warning information. In addition, the output interface 540 may further include a voice output module, a haptic module, and the like in addition to the display device and provide the bio-information estimation result to the user through non-visual methods such as voice, vibration, tactile sensation, and the like.

The communication interface 550 may be connected to an external device and transmit/receive various types data using wired/wireless communication technology under the control of the processor 520. The external device may include an information processing device, such as a smartphone, a tablet personal computer (PC), a desktop PC, a notebook PC, cloud, and the like. For example, the communication interface 540 may receive light signal data measured by a sensor of the external device, transmit the received light signal data to the processor 520, and transmit an estimated bio-information value measured by the processor 520 to the external device.

The communication interface 550 may communicate with the external device by using various wired/wireless communication technology such as Bluetooth communication, Bluetooth low energy (BLE) communication, near field communication (NFC), wireless local access network (WLAN) communication, ZigBee communication, infrared data association (IrDA) communication, Wi-Fi Direct (WFD) communication, ultra-wideband (UWB) communication, Ant+ communication, Wi-Fi communication, radio frequency identification (RFID) communication, 3G communication, 4G communication, 5G communication, and 6G communication. However, the embodiment is not limited thereto.

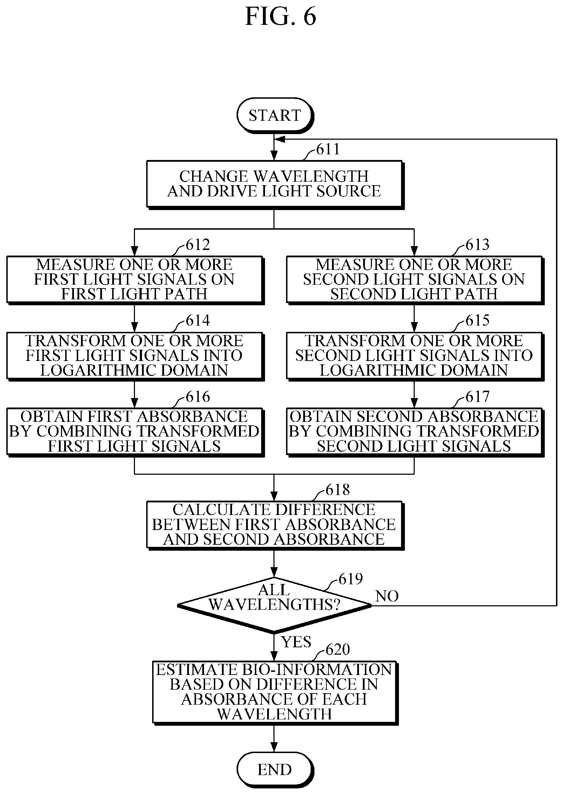

FIG. 6 is a flowchart illustrating a method of estimating bio-information such as an antioxidant level.

The method illustrated in FIG. 6 is an example of a method performed by the apparatuses 100 and 500 for estimating bio-information shown in FIGS. 1 and 5, and will be briefly described below to avoid redundancy. Among operations to be described below, operation 612 to operation 617 may be simultaneously performed irrespective of the order of description. Alternatively, operations 612, 614, and 615 regarding a first light path may be first performed, and thereafter operations 613, 615, and 617 regarding a second light path may be performed, or vice versa.

First, a light source of a previous wavelength may be turned off and a light source of a wavelength to be driven next may be turned on in operation 611. The light source may be provided in plural to emit light of a plurality of different wavelengths and may be sequentially driven in a predetermined order. The wavelength of the light source may range from 400 nm to 600 nm.

Then, first light signals for the driven wavelength are measured using first detectors disposed on the first light path in operation 612, a logarithmic operation is applied to transform first light quantities of the measured first light signals into a logarithmic domain in operation 614, and a first absorbance is obtained by combining the first light quantities transformed into a logarithmic domain in operation 616. The first detectors may be disposed at a first distance from the light source to have the first light path. The logarithmic operation may include common logarithms, natural logarithms, and the like, and is not limited thereto. The combination may be one of an arithmetic mean, a geometric mean, and a harmonic mean, but is not limited thereto.

Similarly, second light signals for the driven wavelength are measured using second detectors disposed on a second light path in operation 613, and a logarithmic operation is applied to transform second light quantities of the measured second light signals into a logarithmic domain in operation 615, and a second absorbance is obtained by combining the second light quantities transformed into a logarithmic domain in operation 617. The second detectors may be disposed at a second distance from the light source to have the second light path.

Thereafter, a difference between the first absorbance and the second absorbance for the wavelength driven in operation 611 is calculated in operation 618. In the process of obtaining the difference between the first absorbance and the second absorbance, the amount of light emitted from the light source is canceled, and thus there is no need to perform a calibration process for obtaining the amount of light emitted from the light source, thereby improving user convenience.

Then, if the next wavelength to be driven remains in operation 619, operations 611 to 618 may be performed for the next wavelength, and if the calculation for all wavelengths is completed in operation 619, bio-information such as an antioxidant level is estimated based on the difference in absorbance of each wavelength obtained in operation 618 in operation 620. For example, an antioxidant peak may be obtained by combining the difference in absorbance of each wavelength and the antioxidant peak may be input to an antioxidant estimation model to obtain an antioxidant level.

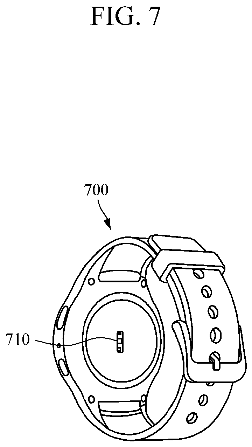

FIGS. 7, 8, and 9 are diagrams illustrating various structures of an electronic device including the apparatus 100 or 500 for estimating bio-information described above.

An electronic device may include a wearable device, for example, a smart watch type, a smart band type, smart eyeglass type, a smart earphone type, a smart ring type, a smart patch, and a smart necklace type, a mobile device, such as a smartphone or a tablet PC, a home appliance, or various types of Internet of Things (IoT) device (e.g., home IoT device).

The electronic device may include a sensor device, a processor, an input device, a communication module, a camera module, an output device, a storage device, and a power module. The components of the electronic device may be integrally mounted in a specific device, or mounted in two or more devices in a distributed manner. The sensor device may include the sensors 110 and 510 of the apparatuses 100 and 500 for estimating bio-information, and may include additional sensors, such as a gyro sensor, and a global positioning system (GPS).

The processor may control the components connected to the processor by executing a program or the like stored in the storage device, and may perform various data processing including bio-information estimation, or operations. The processor may include a main processor, such as a central processing unit and an application processor, and a co-processor that can be operated independently or together with the main processor, for example, a graphics processing unit, an image signal processor, a sensor hub processor, and a communication processor.

The input device may receive a command and/or data to be used in each component of the electronic device from the user or the like. The input device may include a microphone, a mouse, a keyboard, and/or a digital pen (e.g., a stylus pen).

The communication module may support the establishment of a direct (wired) communication channel and/or wireless communication channel between the electronic device and another electronic device or a server in a network environment or the sensor module and the communication therebetween through the established communication channel. The communication module may be operated independently of the processor 920 and may include one or more communication processors that support direct communication and/or wireless communication. The communication module may include a wireless communication module, such as, a cellular communication module, a short-range wireless communication module, a global navigation satellite system (GNSS) communication module, or the like, and/or a wired communication module, such as a local area network (LAN) communication module, a power line communication module, or the like. Such various types of communication modules may be integrated into a single chip, or may be implemented as a plurality of separate chips. The wireless communication module may verify and authenticate the electronic device in a communication network using subscriber information (e.g., international mobile subscriber identity (IMSI)) stored in a subscriber identity module.

The camera module may capture still images and moving images. The camera module may include a lens assembly including one or more lenses, image sensors, image signal processors, and/or flashes. The lens assembly included in the camera module may collect light emitted from a subject to be imaged.

The output device may output data generated or processed by the electronic device in a visual/non-visual manner. The output device may include a sound output device, a display device, an audio module, and/or a haptic module.

The sound output device may output a sound signal to the outside of the electronic device. The sound output device may include a speaker and/or a receiver. The speaker may be used for general purposes, such as multimedia playback or recording playback, and the receiver may be used to receive incoming calls. The receiver may be combined as part of the speaker or may be implemented as an independent separate device.

The display device may visually provide information to the outside of the electronic device. The display device may include a display, a hologram device, or a projector, and control circuitry to control a corresponding one of the display, hologram device, and projector. The display device may include touch circuitry adapted to detect a touch, or sensor circuitry (e.g., a pressure sensor) adapted to measure the intensity of force incurred by the touch.

The audio module may transform a sound into an electrical signal and vice versa. The audio module may obtain the sound via the input device, or output the sound via the sound output device or a speaker and/or a headphone of an external electronic device directly (e.g., wiredly) or wirelessly coupled with the electronic device.

The haptic module may transform an electrical signal into a mechanical stimulus (e.g., a vibration or motion) or electrical stimulus which may be recognized by a user via his or her tactile sensation or kinesthetic sensation. The haptic module may include, for example, a motor, a piezoelectric element, and/or an electric stimulator.

The storage device may store driving conditions required for driving the sensor module and various data required by other components of the electronic device, for example, software and input data and/or output data for commands related to the software. The storage device may include volatile memory and/or non-volatile memory.

The power module may manage power supplied to the electronic device. The power module may be configured as part of a power management integrated circuit (PMIC). The power module may include a battery and the battery may include a non-rechargeable primary cell, a rechargeable secondary cell, and/or a fuel cell.

Referring to FIG. 7, the electronic device may be implemented as a smart watch 700 and may include a body and a wrist strap. A display may be provided on a front surface of the main body, and various application screens containing time information, received message information, and the like may be displayed thereon. A sensor 710 may be disposed on the rear surface of the main body.

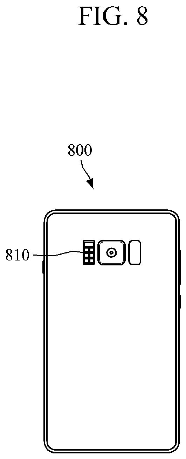

Referring to FIG. 8, the electronic device may be implemented as a mobile device 800 such as a smartphone. The mobile device 800 may include a housing and a display panel. The housing may form the outer appearance of the mobile device 800. The display panel and cover glass may be sequentially arranged on a first surface of the main body, and the display panel may be exposed to the outside through the cover glass. A sensor 810, a camera module, and/or an infrared sensor may be disposed on the rear surface or lateral surface of the housing. A processor and other various components may be disposed inside the housing.

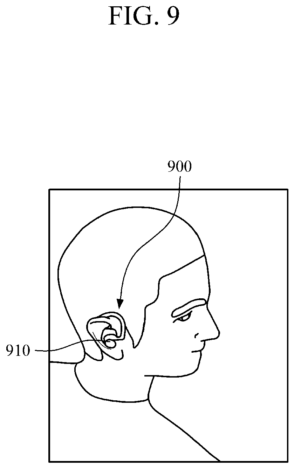

Referring to FIG. 9, the electronic device may be implemented as an ear-wearable device 900. The ear-wearable device 900 may include a main body and an ear strap. The user may wear the electronic device by wearing the ear strap on the auricle. The ear strap may be omitted depending on the shape of the ear-wearable device 900. The main body may be inserted into the external auditory meatus of the user. A sensor device 910 and a processor are disposed in the main body and the process may estimate bio-information using light signals measured by the sensor device 910. Alternatively, the ear-wearable device 900 may cooperate with an external device and transmit the light signals measured by the sensor device 910 to the external device through a communication module, thereby allowing a processor of the external device to estimate bio-information. In this case, an estimated bio-information value may be visually output through a display of the external device, and optionally, may be output through a sound output module provided in the main body of the ear-wearable device.

The disclosed examples may be realized as a computer-readable code written on a computer-readable recording/storage medium. The computer-readable recording/storage medium may be any type of recording device in which data is stored in a computer-readable manner.

Examples of the computer-readable recording/storage medium include a ROM, a RAM, a compact disc (CD)-ROM, a magnetic tape, a floppy disc, an optical data storage, and a carrier wave (e.g., data transmission through the Internet). The computer-readable recording/storage medium may be distributed over a plurality of computer systems connected to a network so that a computer-readable code is written thereto and executed therefrom in a decentralized manner. Functional programs, codes, and code segments for realizing the embodiments can be easily deduced by programmers of ordinary skill in the art, to which the embodiments pertain.

Although various examples have been described, it will be understood that various modifications may be made. The present invention is defined by the appended claims.

## Claims

1. An apparatus for estimating an antioxidant level, the apparatus comprising:
a sensor (110, 510) comprising at least one light source (111, 511) configured to emit light to an object, and at least one first detector configured to detect at least one first light signal scattered or reflected by the object, and at least one second detector configured to detect at least one second light signal scattered or reflected by the object, each of the at least one first light signal having a first light path from the object and each of the at least one second light signal having a second light path from the object that is different from the first light path; and
a processor (120, 520) configured to:
based on the at least one first light signal being detected, obtain (616) a first absorbance by combining first light quantities of the first light path for each wavelength based on the at least one first light signal, wherein obtaining (616) the first absorbance comprises transforming each of the at least one first light signal into a first logarithmic domain and combining the transformed at least one first light signal to obtain (616) the first absorbance,
based on the at least one second light signal being detected, obtain (617) a second absorbance by combining second light quantities of the second light path for each wavelength based on the at least one second light signal, wherein obtaining (617) the second absorbance comprises transforming each of the at least one second light signal into a second logarithmic domain and combining the transformed at least one second light signal to obtain (617) the second absorbance, and
estimate (620) the antioxidant level based on a difference between the first absorbance and the second absorbance for each wavelength without performing a calibration process for measuring the light emitted by the at least one light source (111, 511).

2. The apparatus of claim 1, wherein the at least one light source (111, 511) is disposed at a center (CP) of the sensor (110, 510),
wherein the at least one first detector comprises a plurality of first detectors arranged in a first concentric circular shape at a first distance from the center (CP) of the sensor (110, 510), and
wherein the at least one second detector comprises a plurality of second detectors arranged in a second concentric circular shape at a second distance from the center (CP) of the sensor (110, 510).

3. The apparatus of claim 1, wherein the at least one first detector is disposed at a center (CP) of the sensor (110, 510),
wherein the at least one light source (111, 511) comprises a plurality of light sources arranged in a first concentric circular shape at a first distance from the center (CP) of the sensor (110, 510), and
wherein the at least one second detector comprises a plurality of second detectors arranged in a second concentric circular shape at a second distance from the first concentric circular shape.

4. The apparatus of claim 1, wherein the at least one second detector is disposed at a center (CP) of the sensor (110, 510),
wherein the at least one light source (111, 511) comprises a plurality of light sources arranged in a second concentric circular shape that is at a second distance from the center (CP) of the sensor (110, 510), and
wherein the at least one first detector arranged in a first concentric circular shape at a first distance from the second concentric circular shape.

5. The apparatus of claim 1, wherein the at least one light source (111, 511) has a wavelength in a range from 400 nm to 600 nm.

6. The apparatus of claim 1 further comprising at least one partition wall disposed between the at least one light source (111, 511) and the at least one first detector or between the at least one light source (111, 511) and the at least one second detector.

7. The apparatus of one of claims 1 to 6, wherein a difference between the first light path and the second light path is between 2 mm and 15 mm.

8. The apparatus of claim 1, wherein the processor (120, 520) is further configured to combine the transformed at least one first light signal and combine the transformed at least one second light signal using at least one of an arithmetic mean, a harmonic mean, or a geometric mean.

9. The apparatus of one of claims 1 to 8, wherein the processor (120, 520) is further configured to:
determine an antioxidant peak based on the difference between the first absorbance and the second absorbance for a plurality of wavelengths, and
obtain the antioxidant level based on the antioxidant peak.

10. A computer-readable storage medium storing instructions that, when executed by a processor (120, 520) of an apparatus for estimating an antioxidant level having a sensor (110, 510) comprising at least one light source (111, 511) configured to emit light to an object, and at least one first detector configured to detect at least one first light signal scattered or reflected by the object, each of the at least one first light signal having a first light path from the object, and at least one second detector configured to detect at least one second light signal scattered or reflected by the object, each of the at least one second light signal having a second light path from the object that is different from the first light path, cause the processor (120, 520) to:
obtain (616) a first absorbance based on the at least one first light signal, wherein for obtaining (616) the first absorbance, the processor (120, 520) is further instructed, based on the at least one first light signal being detected, to transform each of the at least one first light signal into a first logarithmic domain and combine the transformed at least one first light signal to obtain the first absorbance;
obtain (617) a second absorbance based on the at least one second light signal, wherein for obtaining the second absorbance, the processor (120, 520) is further instructed, based on the at least one second light signal being detected, to transform each of the at least one second light signal into a second logarithmic domain and combine the transformed at least one second light signal to obtain the second absorbance; and
estimate (620) the antioxidant level based on a difference between the first absorbance and the second absorbance for each wavelength without performing a calibration process for measuring the light emitted by the at least one light source (111, 511).

11. The computer-readable storage medium of claim 10, wherein the processor (120, 520) is further instructed to:
determine an antioxidant peak based on the difference between the first absorbance and the second absorbance for a plurality of wavelengths, and
obtain the antioxidant level based on the antioxidant peak.

12. A method of operating a device worn on a body part of a user for estimating an antioxidant level of the user, the method comprising:
emitting, by a light source (111, 511), light to the body part;
detecting, by a first detector spaced apart from the light source (111, 511), at least one first light signal scattered or reflected by the body part from the light emitted by the light source (111, 511), each of the at least one first light signal having a first light path from the body part;
detecting, by a second detector spaced further apart from the light source (111, 511) than the first detector, at least one second light signal scattered or reflected by the body part from the light emitted by the light source (111, 511), each of the at least one second light signal having a second light path from the body part
that is different from the first light path;
based on the at least one first light signal being detected, obtaining (616), by a processor (120, 520), a first absorbance by combining first light quantities of the first light path for each wavelength based on the at least one first light signal, wherein obtaining (616) the first absorbance comprises transforming each of the at least one first light signal into a first logarithmic domain and combining the transformed at least one first light signal to obtain (616) the first absorbance;
based on the at least one second light signal being detected, obtaining (617), by the processor (120, 520), a second absorbance by combining second light quantities of the second light path for each wavelength based on the at least one second light signal, wherein obtaining (617) the second absorbance comprises transforming each of the at least one second light signal into a second logarithmic domain and combining the transformed at least one second light signal to obtain (617) the second absorbance; and
estimating, by the processor (120, 520), the antioxidant level of the user based on a difference between the first absorbance and the second absorbance for each wavelength without performing a calibration process for measuring the light emitted by the light source (111, 511).

13. The method of claim 12, wherein operating the device comprises operating a smart watch configured to be worn on a wrist of the user.

## Patentansprüche

1. Vorrichtung zum Schätzen eines Antioxidationsmittelgehalts, wobei die Vorrichtung umfasst:
einen Sensor (110, 510), umfassend mindestens eine Lichtquelle (111, 511), die konfiguriert ist, Licht auf ein Objekt zu emittieren, und mindestens einen ersten Detektor, der konfiguriert ist, mindestens ein erstes Lichtsignal zu erfassen, das von dem Objekt gestreut oder reflektiert wird, und mindestens einen zweiten Detektor, der konfiguriert ist, mindestens ein zweites Lichtsignal zu erfassen, das von dem Objekt gestreut oder reflektiert wird, wobei jedes des mindestens einen ersten Lichtsignals einen ersten Lichtweg von dem Objekt aufweist und jedes des mindestens einen zweiten Lichtsignals einen zweiten Lichtweg von dem Objekt aufweist, der sich von dem ersten Lichtweg unterscheidet; und
einen Prozessor (120, 520), der konfiguriert ist zum:
basierend darauf, dass das mindestens eine erste Lichtsignal erfasst wird, Erhalten (616) einer ersten Absorbanz durch Kombinieren erster Lichtmengen des ersten Lichtwegs für jede Wellenlänge basierend auf dem mindestens einen ersten Lichtsignal, wobei das Erhalten (616) der ersten Absorbanz das Transformieren jedes des mindestens einen ersten Lichtsignals in einen ersten logarithmischen Bereich und das Kombinieren des transformierten mindestens einen ersten Lichtsignals umfasst, um die erste Absorbanz zu erhalten;
basierend darauf, dass das mindestens eine zweite Lichtsignal erfasst wird, Erhalten (617) einer zweiten Absorbanz durch Kombinieren zweiter Lichtmengen des zweiten Lichtwegs für jede Wellenlänge basierend auf dem mindestens einen zweiten Lichtsignal, wobei das Erhalten (617) der zweiten Absorbanz das Transformieren jedes des mindestens einen zweiten Lichtsignals in einen zweiten logarithmischen Bereich und das Kombinieren des transformierten mindestens einen zweiten Lichtsignals umfasst, um die zweite Absorbanz zu erhalten; und
Schätzen (620) des Antioxidationsmittelgehalts basierend auf einer Differenz zwischen der ersten Absorbanz und der zweiten Absorbanz für jede Wellenlänge, ohne einen Kalibrierungsprozess zum Messen des von der mindestens einen Lichtquelle (111, 511) emittierten Lichts durchzuführen.

2. Vorrichtung nach Anspruch 1, wobei die mindestens eine Lichtquelle (111, 511) in einer Mitte (CP) des Sensors (110, 510) angeordnet ist,
wobei der mindestens eine erste Detektor eine Vielzahl erster Detektoren umfasst, die in einer ersten konzentrischen Kreisform in einem ersten Abstand von der Mitte (CP) des Sensors (110, 510) angeordnet sind, und
wobei der mindestens eine zweite Detektor eine Vielzahl zweiter Detektoren umfasst, die in einer zweiten konzentrischen Kreisform in einem zweiten Abstand von der Mitte (CP) des Sensors (110, 510) angeordnet sind.

3. Vorrichtung nach Anspruch 1, wobei der mindestens eine erste Detektor in einer Mitte (CP) des Sensors (110, 510) angeordnet ist,
wobei die mindestens eine Lichtquelle (111, 511) eine Vielzahl von Lichtquellen umfasst, die in einer ersten konzentrischen Kreisform in einem ersten Abstand von der Mitte (CP) des Sensors (110, 510) angeordnet sind, und
wobei der mindestens eine zweite Detektor eine Vielzahl zweiter Detektoren umfasst, die in einer zweiten konzentrischen Kreisform in einem zweiten Abstand von der ersten konzentrischen Kreisform angeordnet sind.

4. Vorrichtung nach Anspruch 1, wobei der mindestens eine zweite Detektor in einer Mitte (CP) des Sensors (110, 510) angeordnet ist,
wobei die mindestens eine Lichtquelle (111, 511) eine Vielzahl von Lichtquellen umfasst, die in einer konzentrischen Kreisform in einem ersten Abstand von der Mitte (CP) des Sensors (110, 510) angeordnet sind, und
wobei der mindestens eine erste Detektor in einer ersten konzentrischen Kreisform in einem ersten Abstand von der ersten konzentrischen Kreisform angeordnet ist.

5. Vorrichtung nach Anspruch 1, wobei die mindestens eine Lichtquelle (111, 511) eine Wellenlänge in einem Bereich von 400 nm bis 600 nm aufweist.

6. Vorrichtung nach Anspruch 1, ferner umfassend mindestens eine Trennwand, die zwischen der mindestens einen Lichtquelle (111, 511) und dem mindestens einen ersten Detektor oder zwischen der mindestens einen Lichtquelle (111, 511) und dem mindestens einen zweiten Detektor angeordnet ist.

7. Vorrichtung nach einem der Ansprüche 1 bis 6, wobei eine Differenz zwischen dem ersten Lichtweg und dem zweiten Lichtweg zwischen 2 mm und 15 mm beträgt.

8. Vorrichtung nach Anspruch 1, wobei der Prozessor (120, 520) ferner konfiguriert ist zum:
Kombinieren des transformierten mindestens einen ersten Lichtsignals und Kombinieren des transformierten mindestens einen zweiten Lichtsignals unter Verwendung mindestens eines von einem arithmetischen Mittel, einem harmonischen Mittel oder einem geometrischen Mittel.

9. Vorrichtung nach einem der Ansprüche 1 bis 8, wobei der Prozessor (120, 520) ferner konfiguriert ist zum:
Bestimmen eines Antioxidationsmittelpeaks basierend auf der Differenz zwischen der ersten Absorbanz und der zweiten Absorbanz für eine Vielzahl von Wellenlängen, und
Erhalten des Antioxidationsmittelgehalts basierend auf dem Antioxidationsmittelpeak.

10. Computerlesbares Speichermedium, das Anweisungen speichert, die bei Ausführung durch einen Prozessor (120, 520) einer Vorrichtung zum Schätzen eines Antioxidationsmittelgehalts einen Sensor (110, 510), umfassend mindestens eine Lichtquelle (111, 511), die konfiguriert ist, Licht auf ein Objekt zu emittieren, und mindestens einen ersten Detektor, der konfiguriert ist, mindestens ein erstes Lichtsignal zu erfassen, das von dem Objekt gestreut oder reflektiert wird, und mindestens einen zweiten Detektor, der konfiguriert ist, mindestens ein zweites Lichtsignal zu erfassen, das von dem Objekt gestreut oder reflektiert wird, wobei jedes des mindestens einen ersten Lichtsignals einen ersten Lichtweg von dem Objekt aufweist und jedes des mindestens einen zweiten Lichtsignals einen zweiten Lichtweg von dem Objekt aufweist, der sich von dem ersten Lichtweg unterscheidet, den Prozessor (120, 520) veranlassen zum:
Erhalten (616) einer ersten Absorbanz basierend auf dem mindestens einen ersten Lichtsignal, wobei zum Erhalten (616) der ersten Absorbanz der Prozessor (120, 520) ferner angewiesen wird, basierend darauf, dass das mindestens eine erste Lichtsignal erfasst wird, jedes des mindestens einen ersten Lichtsignals in einen ersten logarithmischen Bereich zu transformieren und das transformierte mindestens eine erste Lichtsignal zu kombinieren, um die erste Absorbanz zu erhalten;
Erhalten (617) einer zweiten Absorbanz basierend auf dem mindestens einen zweiten Lichtsignal, wobei zum Erhalten (617) der zweiten Absorbanz der Prozessor (120, 520) ferner angewiesen wird, basierend darauf, dass das mindestens eine zweite Lichtsignal erfasst wird, jedes des mindestens einen zweiten Lichtsignals in einen zweiten logarithmischen Bereich zu transformieren und das transformierte mindestens eine zweite Lichtsignal zu kombinieren, um die zweite Absorbanz zu erhalten; und
Schätzen (620) des Antioxidationsmittelgehalts basierend auf einer Differenz zwischen der ersten Absorbanz und der zweiten Absorbanz für jede Wellenlänge, ohne einen Kalibrierungsprozess zum Messen des von der mindestens einen Lichtquelle (111, 511) emittierten Lichts durchzuführen.

11. Computerlesbares Speichermedium nach Anspruch 10, wobei der Prozessor (120, 520) ferner angewiesen wird zum:
Bestimmen eines Antioxidationsmittelpeaks basierend auf der Differenz zwischen der ersten Absorbanz und der zweiten Absorbanz für eine Vielzahl von Wellenlängen, und
Erhalten des Antioxidationsmittelgehalts basierend auf dem Antioxidationsmittelpeak.

12. Verfahren zum Betreiben einer an einem Körperteil eines Benutzers getragenen Vorrichtung zum Schätzen eines Antioxidationsmittelgehalts des Benutzers, wobei das Verfahren umfasst:
Emittieren, durch eine Lichtquelle (111, 511), von Licht auf den Körperteil;
Erfassen, durch einen ersten Detektor, der von der Lichtquelle (111, 511) beabstandet ist, mindestens eines ersten Lichtsignals, das von dem Körperteil gestreut oder reflektiert wird, wobei jedes des mindestens einen ersten Lichtsignals einen ersten Lichtweg von dem Körperteil aufweist;
Erfassen, durch einen zweiten Detektor, der weiter von der Lichtquelle (111, 511) beabstandet ist als der erste Detektor, mindestens eines zweiten Lichtsignals, das von dem Körperteil gestreut oder reflektiert wird, wobei jedes des mindestens einen zweiten Lichtsignals einen zweiten Lichtweg von dem Körperteil aufweist, der sich von dem ersten Lichtweg unterscheidet;
basierend darauf, dass das mindestens eine erste Lichtsignal erfasst wird, Erhalten (616), durch einen Prozessor (120, 520), einer ersten Absorbanz durch Kombinieren erster Lichtmengen des ersten Lichtwegs für jede Wellenlänge basierend auf dem mindestens einen ersten Lichtsignal, wobei das Erhalten (616) der ersten Absorbanz das Transformieren jedes des mindestens einen ersten Lichtsignals in einen ersten logarithmischen Bereich und das Kombinieren des transformierten mindestens einen ersten Lichtsignals umfasst, um die erste Absorbanz zu erhalten;
basierend darauf, dass das mindestens eine zweite Lichtsignal erfasst wird, Erhalten (617), durch den Prozessor (120, 520), einer zweiten Absorbanz durch Kombinieren zweiter Lichtmengen des zweiten Lichtwegs für jede Wellenlänge basierend auf dem mindestens einen zweiten Lichtsignal, wobei das Erhalten (617) der zweiten Absorbanz das Transformieren jedes des mindestens einen zweiten Lichtsignals in einen zweiten logarithmischen Bereich und das Kombinieren des transformierten mindestens einen zweiten Lichtsignals umfasst, um die zweite Absorbanz zu erhalten; und
Schätzen, durch den Prozessor (120, 520), des Antioxidationsmittelgehalts des Benutzers basierend auf einer Differenz zwischen der ersten Absorbanz und der zweiten Absorbanz für jede Wellenlänge, ohne einen Kalibrierungsprozess zum Messen des von der Lichtquelle (111, 511) emittierten Lichts durchzuführen.

13. Verfahren nach Anspruch 12, wobei das Betreiben der Vorrichtung das Betreiben einer Smartwatch umfasst, die konfiguriert ist, am Handgelenk des Benutzers getragen zu werden.

## Revendications

1. Appareil d'estimation d'un niveau d'antioxydant, l'appareil comprenant :
un capteur (110, 510) comprenant au moins une source de lumière (111, 511) configurée pour émettre de la lumière sur un objet, et au moins un premier détecteur configuré pour détecter au moins un premier signal lumineux diffusé ou réfléchi par l'objet, et au moins un second détecteur configuré pour détecter au moins un second signal lumineux diffusé ou réfléchi par l'objet, chacun de l'au moins un premier signal lumineux ayant un premier trajet lumineux depuis l'objet et chacun de l'au moins un second signal lumineux ayant un second trajet lumineux depuis l'objet qui est différent du premier trajet lumineux ; et
un processeur (120, 520) configuré pour :
sur la base de la détection de l'au moins un premier signal lumineux, obtenir (616) une première absorbance en combinant des premières quantités de lumière du premier trajet lumineux pour chaque longueur d'onde sur la base de l'au moins un premier signal lumineux, dans lequel l'obtention (616) de la première absorbance comprend la transformation de chacun de l'au moins un premier signal lumineux dans un premier domaine logarithmique et la combinaison de l'au moins un premier signal lumineux transformé pour obtenir la première absorbance ;
sur la base de la détection de l'au moins un second signal lumineux, obtenir (617) une seconde absorbance en combinant des secondes quantités de lumière du second trajet lumineux pour chaque longueur d'onde sur la base de l'au moins un second signal lumineux, dans lequel l'obtention (617) de la seconde absorbance comprend la transformation de chacun de l'au moins un second signal lumineux dans un second domaine logarithmique et la combinaison de l'au moins un second signal lumineux transformé pour obtenir la seconde absorbance ; et
estimer (620) le niveau d'antioxydant sur la base d'une différence entre la première absorbance et la seconde absorbance pour chaque longueur d'onde sans effectuer un processus d'étalonnage pour mesurer la lumière émise par l'au moins une source de lumière (111, 511).

2. Appareil selon la revendication 1, dans lequel l'au moins une source de lumière (111, 511) est disposée au centre (CP) du capteur (110, 510),
dans lequel l'au moins un premier détecteur comprend une pluralité de premiers détecteurs disposés selon une première forme circulaire concentrique à une première distance du centre (CP) du capteur (110, 510), et
dans lequel l'au moins un second détecteur comprend une pluralité de seconds détecteurs disposés selon une seconde forme circulaire concentrique à une seconde distance du centre (CP) du capteur (110, 510).

3. Appareil selon la revendication 1, dans lequel l'au moins un premier détecteur est disposé au centre (CP) du capteur (110, 510),
dans lequel l'au moins une source de lumière (111, 511) comprend une pluralité de sources de lumière disposées selon une première forme circulaire concentrique à une première distance du centre (CP) du capteur (110, 510), et
dans lequel l'au moins un second détecteur comprend une pluralité de seconds détecteurs disposés selon une seconde forme circulaire concentrique à une seconde distance de la première forme circulaire concentrique.

4. Appareil selon la revendication 1, dans lequel l'au moins un second détecteur est disposé au centre (CP) du capteur (110, 510),
dans lequel l'au moins une source de lumière (111, 511) comprend une pluralité de sources de lumière disposées selon une forme circulaire concentrique à une première distance du centre (CP) du capteur (110, 510), et
dans lequel l'au moins un premier détecteur est disposé selon une première forme circulaire concentrique à une première distance de la première forme circulaire concentrique.

5. Appareil selon la revendication 1, dans lequel l'au moins une source de lumière (111, 511) a une longueur d'onde dans une plage de 400 nm à 600 nm.

6. Appareil selon la revendication 1, comprenant en outre au moins une paroi de séparation disposée entre l'au moins une source de lumière (111, 511) et l'au moins un premier détecteur ou entre l'au moins une source de lumière (111, 511) et l'au moins un second détecteur.

7. Appareil selon l'une des revendications 1 à 6, dans lequel une différence entre le premier trajet lumineux et le second trajet lumineux est comprise entre 2 mm et 15 mm.

8. Appareil selon la revendication 1, dans lequel le processeur (120, 520) est en outre configuré pour :
combiner l'au moins un premier signal lumineux transformé et combiner l'au moins un second signal lumineux transformé en utilisant au moins l'une parmi une moyenne arithmétique, une moyenne harmonique ou une moyenne géométrique.

9. Appareil selon l'une des revendications 1 à 8, dans lequel le processeur (120, 520) est en outre configuré pour :
déterminer un pic d'antioxydant sur la base de la différence entre la première absorbance et la seconde absorbance pour une pluralité de longueurs d'onde, et
obtenir le niveau d'antioxydant sur la base du pic d'antioxydant.

10. Support de stockage lisible par ordinateur stockant des instructions qui, lorsqu'elles sont exécutées par un processeur (120, 520) d'un appareil d'estimation d'un niveau d'antioxydant comprenant un capteur (110, 510) comprenant au moins une source de lumière (111, 511) configurée pour émettre de la lumière sur un objet, et au moins un premier détecteur configuré pour détecter au moins un premier signal lumineux diffusé ou réfléchi par l'objet, et au moins un second détecteur configuré pour détecter au moins un second signal lumineux diffusé ou réfléchi par l'objet, chacun de l'au moins un premier signal lumineux ayant un premier trajet lumineux depuis l'objet et chacun de l'au moins un second signal lumineux ayant un second trajet lumineux depuis l'objet qui est différent du premier trajet lumineux, amènent le processeur (120, 520) à :
obtenir (616) une première absorbance sur la base de l'au moins un premier signal lumineux, dans lequel, pour obtenir (616) la première absorbance, le processeur (120, 520) reçoit en outre pour instruction, sur la base de la détection de l'au moins un premier signal lumineux, de transformer chacun de l'au moins un premier signal lumineux dans un premier domaine logarithmique et de combiner l'au moins un premier signal lumineux transformé pour obtenir la première absorbance ;
obtenir (617) une seconde absorbance sur la base de l'au moins un second signal lumineux, dans lequel, pour obtenir (617) la seconde absorbance, le processeur (120, 520) reçoit en outre pour instruction, sur la base de la détection de l'au moins un second signal lumineux, de transformer chacun de l'au moins un second signal lumineux dans un second domaine logarithmique et de combiner l'au moins un second signal lumineux transformé pour obtenir la seconde absorbance ; et
estimer (620) le niveau d'antioxydant sur la base d'une différence entre la première absorbance et la seconde absorbance pour chaque longueur d'onde sans effectuer un processus d'étalonnage pour mesurer la lumière émise par l'au moins une source de lumière (111, 511).

11. Support de stockage lisible par ordinateur selon la revendication 10, dans lequel le processeur (120, 520) reçoit en outre pour instruction de :
déterminer un pic d'antioxydant sur la base de la différence entre la première absorbance et la seconde absorbance pour une pluralité de longueurs d'onde, et
obtenir le niveau d'antioxydant sur la base du pic d'antioxydant.

12. Procédé de fonctionnement d'un dispositif porté sur une partie du corps d'un utilisateur pour estimer un niveau d'antioxydant de l'utilisateur, le procédé comprenant :
l'émission, par une source de lumière (111, 511), de lumière sur la partie du corps ;
la détection, par un premier détecteur espacé de la source de lumière (111, 511), d'au moins un premier signal lumineux diffusé ou réfléchi par la partie du corps, chacun de l'au moins un premier signal lumineux ayant un premier trajet lumineux depuis la partie du corps ;
la détection, par un second détecteur espacé davantage de la source de lumière (111, 511) que le premier détecteur, d'au moins un second signal lumineux diffusé ou réfléchi par la partie du corps, chacun de l'au moins un second signal lumineux ayant un second trajet lumineux depuis la partie du corps qui est différent du premier trajet lumineux ;
sur la base de la détection de l'au moins un premier signal lumineux, l'obtention (616), par un processeur (120, 520), d'une première absorbance en combinant des premières quantités de lumière du premier trajet lumineux pour chaque longueur d'onde sur la base de l'au moins un premier signal lumineux, dans lequel l'obtention (616) de la première absorbance comprend la transformation de chacun de l'au moins un premier signal lumineux dans un premier domaine logarithmique et la combinaison de l'au moins un premier signal lumineux transformé pour obtenir la première absorbance ;
sur la base de la détection de l'au moins un second signal lumineux, l'obtention (617), par le processeur (120, 520), d'une seconde absorbance en combinant des secondes quantités de lumière du second trajet lumineux pour chaque longueur d'onde sur la base de l'au moins un second signal lumineux, dans lequel l'obtention (617) de la seconde absorbance comprend la transformation de chacun de l'au moins un second signal lumineux dans un second domaine logarithmique et la combinaison de l'au moins un second signal lumineux transformé pour obtenir la seconde absorbance ; et
l'estimation, par le processeur (120, 520), du niveau d'antioxydant de l'utilisateur sur la base d'une différence entre la première absorbance et la seconde absorbance pour chaque longueur d'onde sans effectuer un processus d'étalonnage pour mesurer la lumière émise par la source de lumière (111, 511).

13. Procédé selon la revendication 12, dans lequel le fonctionnement du dispositif comprend le fonctionnement d'une montre intelligente configurée pour être portée au poignet de l'utilisateur.
